# EUROPÄISCHE PATENTSCHRIFT

(11) **EP 2 397 834 B1**
(45) Veröffentlichungstag und Bekanntmachung des Hinweises auf die Patenterteilung: **23.01.2019**
(21) Anmeldenummer: 11001762.1
(22) Anmeldetag: 03.03.2011
(51) Int. Cl.: G01N 1/12, G01N 33/20

(54) **Messsonden zur Messung und Probennahme mit einer Metallschmelze**
Measuring probes for measuring and sampling with a metal melt
Sondes de mesure pour la mesure et le prélèvement d'échantillon à l'aide d'une pièce en fonte métallique

(30) Priorität: 18.06.2010 DE 102010024282
(43) Veröffentlichungstag der Anmeldung: 21.12.2011
(73) Patentinhaber: Heraeus Electro-Nite International N.V., 3530 Houthalen (BE)
(72) Erfinder: Beyens, Dries, 3640 Kinrooi (BE); Neyens, Guido Jacobus, 3680 Opoeteren (BE)
(74) Vertreter: Heraeus IP

(56) Entgegenhaltungen:
- DE-A1- 3 000 201
- JP-A- 7 306 196
- US-A- 4 007 641
- US-A1- 2005 132 823
- US-A1- 2007 137 286
- US-A1- 2007 137 324

## Beschreibung

Die Erfindung betrifft Messsonden zur Messung und Probennahme mit einer Metallschmelze mit einem an einer Lanze angeordneten Messkopf, wobei der Messkopf mindestens einen Temperatursensor und eine Probenkammer trägt, wobei die Probenkammer von dem Messkopf umgeben ist und einen durch den Messkopf hindurch verlaufenden Einlaufkanal aus einem Quarzglasrohr aufweist.

Derartige Messsonden sind grundsätzlich bekannt und werden unter anderem eingesetzt bei der Stahlherstellung in sogenannten Konvertern oder in Lichtbogenöfen.

In einem Konverter (sogenannter BOF-Konverter - fachübliche Abkürzung für die englische Bezeichnung Basic Oxygen Furnace / Basis-Sauerstoff-Ofen) wird mittels einer Lanze Sauerstoff in die Metallschmelze eingeblasen. Der Konverter ist mit feuerfestem Material ausgekleidet, das der Erosion durch Schlacke und Wärme während des Sauerstoff-Blasprozesses optimal widersteht. In den Konverter wird Schrott und Kalk (Kalziumoxid) zugegeben, um die Schmelze zu kühlen und Phosphor, Silizium und Mangan zu entfernen. Der Sauerstoff verbrennt den Kohlenstoff zu Kohlenmonoxid und Kohlendioxid. Mangan, Silizium und Phosphor werden oxidiert und mit Kalziumoxid und Eisenoxid zu Schlacke umgewandelt. Da diese Oxidationsreaktion hochexothermisch verläuft, muss der Prozess gekühlt werden, um die Temperatur der Schmelze zu regeln. Die Kühlung erfolgt durch Zugabe von Schrott und Eisenerz während des Blasprozesses. Der Sauerstoff-Blasprozess selbst benötigt etwa 15 - 20 Minuten, unabhängig von der Größe des Konverters, die etwa 70 - 400 Tonnen betragen kann. Die Sauerstoffdurchflussrate der Lanze ist dabei an die Größe des Konverters bzw. das Gewicht der Schmelze angepasst. Das Laden und Beladen von Stahl und Schlacke, einschließlich der Temperaturmessung und Probennahme zur Analyse der Schmelze führt dann zu einer Zeitdauer zwischen 2 Abstichen von 40 - 60 Minuten. Der Gesamtprozess ist gekennzeichnet durch eine hohe Produktivität und führt zu einem Stahl mit einem geringen Gehalt an Verunreinigungen. Der Abstich erfolgt durch Neigen des Ofens durch eine Abstichloch in eine Gießpfanne. Während dieser Operation werden Eisenlegierungen zur Regelung der Stahlzusammensetzung in die Gießpfanne gegeben. Eine wichtige Entwicklung in der Technik in der Sauerstoff-Blaslanzen besteht darin, dass inertes Gas, üblicherweise Argon, durch den Konverterboden der Schmelze zugefügt wird, um die Schmelze und die Schlacke zu rühren. Durch diesen Prozess wird die Effizienz deutlich erhöht, der Eisenverlust und der Phosphorgehalt sinkt. Des Weiteren wird die Wärme- und das Massengleichgewicht des Prozesses verbessert und damit werden die Kosten reduziert.

Messsonden zur Verwendung im Konverter sind beispielsweise in DE 10 2005 060 492 und DE 10 2005 060 493 beschrieben.

In einem Lichtbogenofen wird Schrott geschmolzen durch die Energie eines elektrischen Lichtbogens, der zwischen den Spitzen von Graphitelektroden und der leitfähigen Schrottbeladung erzeugt wird. Die 3 Elektroden und das Ofendach werden zur Beladung des Ofens mit Schrott angehoben, um eine Einfüllöffnung freizugeben. Die Elektroden halten den Lichtbogen entsprechend der vorgewählten Spannung und des vorgewählten Stroms aufrecht, so dass die für das Schmelzen und Frischen benötigte Energie zur Verfügung steht. Lichtbogenöfen haben einen inneren Durchmesser von etwa 6 - 9 Meter und eine Kapazität von 100 - 200 Tonnen Stahl. Die Zeit zwischen 2 Abstichen in diesen Öfen beträgt etwa 90 - 110 Minuten.

Messsonden zum Einsatz in Lichtbogenöfen sind beispielsweise aus DE 28 45 566, DE 32 03 505 oder DE 103 60 625 bekannt.

US2007/0137324 beschreibt eine Messsonde zur Probenentnahme von flüssigem Metall. Auf das Problem der Gasblasenbildung wird darin nicht eingegangen.

US4,007,641 beschreibt ebenfalls eine Sonde zur Entnahme von flüssigem Metall, dabei wird in der Probenkammer ein Unterdruck erzeugt, um die Bildung von Gasblasen in der Probe zu minimieren.

Zur Überwachung der Prozesse im Konverter oder im Lichtbogenofen ist es notwendig, zur Probennahme eine vollständige Füllung der Probenkammer einer Messsonde bei relativ geringen Temperaturen zu erreichen, wobei Gasblasen in der Probe vermieden werden sollen. Solche Probenahme nicht immer einfach, insbesondere während des Blasprozesses im Konverter, da die theoretische Dichte der Stahlschmelze stark schwankt wegen des Sauerstoffblasprozesses von oben einerseits und des Einblasens von Inertgas durch den Konverterboden andererseits. Des Weiteren tendiert die Industrie zur Verwendung solcher Öfen, die eine nur geringe Überhitzung der Schmelze (also eine geringe Differenz zwischen der Badtemperatur und der Liquidustemperatur) gestatten.

Der Erfindung liegt daher die Aufgabe zugrunde, die vorhandenen Messsonden und Probennehmer zu verbessern und eine weitestgehend gasfreie Probennahme zu ermöglichen, also die Probenqualität zu verbessern. Vorzugsweise soll auch die Entnahme der Probe aus der Messsonde vereinfacht werden.

Erfindungsgemäß wird die Aufgabe gelöst durch die Merkmale der unabhängigen Ansprüche. Vorteilhafte Ausgestaltungen sind in den Unteransprüchen angegeben.

Es hat sich gezeigt, dass eine Messsonde zur Messung und Probennahme in einer Metallschmelze mit einem an einer Lanze angeordneten Messkopf, wobei der Messkopf mindestens einen Temperatursensor und eine Probenkammer trägt, wobei die Probenkammer zumindest teilweise von dem Messkopf umgeben ist und einen durch den Messkopf hindurch verlaufenden Einlaufkanal aufweist, der vorzugsweise aus einem Quarzglasrohr gebildet ist, dann ausgezeichnete, gasblasenfreie Proben ermöglicht, wenn die Länge L eines Bereichs des Quarzglasrohres, der in dem Messkopf verläuft ein Verhältnis zum Quadrat eines Mindestdurchmessers D, den das Quarzglasrohr an mindestens einer Stelle in diesem inneren Bereich aufweist, von L/D² < 0,6 mm⁻¹ aufweist, wobei dieses Verhältnis vorzugsweise < 0,45 mm⁻¹ und insbesondere vorzugsweise < 0,3 mm⁻¹ ist. Bei geringer Überhitzung der Metallschmelze hat sich ein geringes Verhältnis als vorteilhaft erwiesen, beispielsweise ein Verhältnis L/D² < 0,6 mm⁻¹ bei einer Überhitzung von > 100°C und ein Verhältnis L/D² < 0,3 mm⁻¹ bei eine Überhitzung von < 80°C. Insbesondere ist es vorteilhaft, wenn das Verhältnis L/D² kleiner als 0,6 mm⁻¹, vorzugsweise kleiner als 0,45 mm⁻¹, besonders bevorzugt kleiner als 0,3 mm⁻¹ und wobei der Gegendruck P_{g} des Messkopfes jeweils kleiner als 20 mbar ist.

Zweckmäßig ist es, dass der Messkopf aus einem Material aus der Gruppe Keramik, Zement, Stahl, Gießereisand gebildet ist. Insbesondere ist es weiterhin vorteilhaft, dass die Probenkammer zumindest teilweise von einem Sandköper aus Gießereisand umgeben ist. Der Messkopf kann weiterhin derart gestaltet sein, dass die Probenkammer in einer ersten und einer zweiten, jeweils senkrecht zueinander angeordneten Richtung jeweils eine größere Länge aufweist in einer dritten, senkrecht zu der ersten und zu der zweiten Richtung angeordneten Richtung und, dass der Einlaufkanal senkrecht zu der dritten Richtung in die Probenkammer mündet. Derart sind sogenannte Flachprobenkammern ausgebildet, die eine kreisrunde oder ovale bzw. längliche Querschnittsfläche und zu dieser senkrecht angeordnet eine kleinere, im Wesentlichen rechteckig ausgebildete Querschnittsfläche aufweisen, wobei die kleinere Querschnittsfläche abgerundete Ecken aufweisen kann. Der Einlaufkanal verläuft also parallel zur größeren und senkrecht zur kleineren Querschnittsfläche. Vorteilhaft ist es weiterhin, dass der Messkopf zusätzlich mindestens einen elektrochemischen Sensor trägt, um eine flexiblere und vielseitigere Nutzung zu ermöglichen und gleichzeitig weitere Parameter der Metallschmelze messen zu können.

Vorteilhaft ist es, dass die Entlüftung der Probenkammer ermöglicht wird. Die Probenkammer besteht voteilhaft aus zwei in bekannter Weise parallel zur Langsachse der Probenkammer trennbaren Halbschalen, welche mit ihren Rändern derart zusammengehalten werden, dass während des Hineinfließens des flüssigen Metalls Luft aus der Probenkammer entweichen kann, flüssiges Metall zwischen den Halbschalen aber nicht austreten kann. Vorteilhaft ist es dass die Probenkammer in einem porösen Sandkörper angeordnet ist, um die Entlüftung zu gewährleisten. Die beide Halbschalen werden durch eine Klammer zusammengepresst und die Probenkammer ist ausreichend im Sandkörper fixiert, so dass sich die beiden Halbschalen infolge des beim Eintauchen in die Schmelze auftretenden ferrostatischen Druckes nicht öffnen. Die Ränder der Halbschalen können beispielsweise mit kleinen Löchern oder Rillen versehen werden, um die Entlüftung der Probenkammer zu ermöglichen, wobei die Bildung von Graten, durch aus der Probenkammer austretenden Schmelze vermieden wird.
Üblicherweise werden die erfindungsgemäßen Messsonden von oben in das die Metallschmelze enthaltende Gefäß eingetaucht. Dieser Tauchvorgang geschieht häufig automatisch, beispielsweise mit einer automatischen Sublanze. Nach der Messung wird die Sublanze mit der Messsonde seitlich aus dem Bereich des die Metallschmelze enthaltenden Gefäßes herausgeschwenkt und abgestoßen. Dabei fällt die Messsonde einige Meter in die Tiefe. Nach dem Aufprall auf den Boden kann die Probe unbeschädigt und einfach aus der Probenkammer entnommen werden.

Erfindungsgemäß werden beschriebene Messsonden zur Messung und Probennahme in einer in einem Konverter zur Stahlschmelze angeordneten Metallschmelze während eines Blasprozesses oder zur Messung und Probennahme in einer in einem Lichtbogenofen angeordneten Metallschmelze verwendet.

Nachfolgend wird die Erfindung beispielhaft anhand einer Zeichnung näher erläutert. In der Zeichnung zeigt:
- Figur 1: eine schematische Darstellung eines Querschnitts durch einen Konverter
- Figur 2: eine schematische Darstellung der erfindungsgemäßen Messsonde mit dem Messkopf
- Figur 3: einen Schnitt durch den erfindungsgemäßen Messkopf
- Figur 4: die schematische Darstellung der Druckmessung am offenen Rohr und
- Figur 5: die schematische Darstellung der Druckmessung am Messkopf.

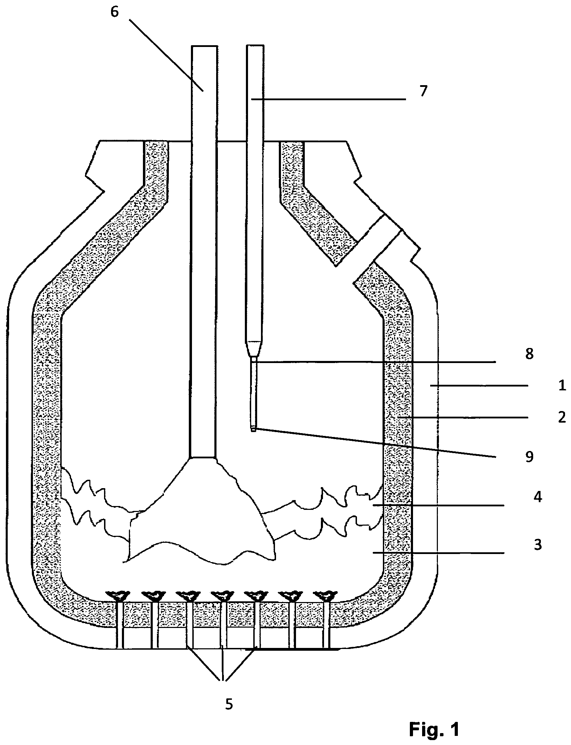

Figur 1 zeigt einen Konverter 1 mit einer Auskleidung 2. In dem Konverter 1 ist eine Stahlschmelze 3 enthalten, auf der eine Schlackeschicht 4 aufliegt. Zur Stahlherstellung wird durch den Boden des Konverters 1 durch Bodendüsen 5 Argon in die Metallschmelze eingeblasen. Von oben wird mittels einer Blaslanze 6 Sauerstoff eingeblasen. Neben der Blaslanze 6 wird eine sogenannte Sublanze 7 in den Konverter 1 eingeführt, an deren Eintauchende eine Messsonde 8 mit einem Messkopf 9 angeordnet ist. Der Messvorgang erfolgt während des Einblasens von Sauerstoff, in der Regel etwa 2 Minuten vor dem Ende des Sauerstoffeinblasens. Dabei wird die Temperatur gemessen und eine Probe zur Bestimmung des Kohlenstoffgehalts genommen. Anhand der Ergebnisse der Messungen kann eine Korrektur des Blasmodels erfolgen, um die Qualität der Stahlschmelze verändern zu können. Nach dem Sauerstoffeinblasen kann eine 2. Messung erfolgen. Dabei wird in der Regel neben der Temperatur der aktive Sauerstoffgehalt in der Stahlschmelze gemessen und es wird eine im Labor auszuwertende Probe zur Bestimmung der endgültigen Zusammensetzung des Stahls genommen. Anhand des Sauerstoffgehaltes kann innerhalb von wenigen Sekunden der aktuelle Kohlenstoffgehalt im Stahl bestimmt werden. Des Weiteren kann eine Vorkalkulation der benötigten Menge eines Deoxidationsmittels (Aluminium) erfolgen.

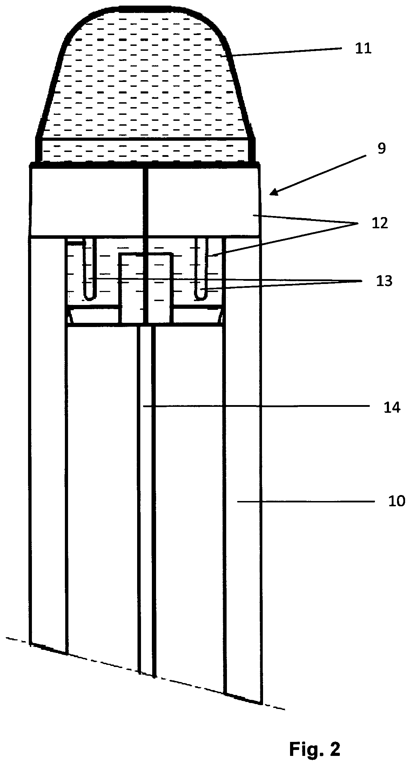

Die in Figur 2 darstellte Messsonde 8 zeigt den am Eintauchende eines Trägerrohres 10 angeordneten Messkopf 9. Der Messkopf 9 weist zum Schutz der Einlauföffnung und der Sensoren eine Kunststoffkappe 11 auf, die während des Durchtritts durch die Schlacke 4 verbrennt und die Sensorik bzw. die Einlauföffnung in die Metallschmelze freigibt. Die Kunststoffkappe 11 kann an ihrer Innenseite durch eine Metallkappe oder Metallschicht ergänzt werden, die aus Stahl gebildet sein kann und sich in der Stahlschmelze, in der die Messsonde eingesetzt wird, auflöst. Der Messkopf 9 weist einen Sandkörper 12 aus Gießereisand auf, welcher Rippen 13 aufweist, mit denen der Sandkörper 12 in das Trägerrohr 10 gepresst wird, um einen festen Halt zu sichern. An dem rückseitigen Ende des Messkopfes 9 sind Verbindungskabel 14 angeordnet, mit denen die von den Sensoren gewonnenen Signale durch das Trägerrohr 10 und die Sublanze 7 hindurch zu einer Auswerteeinheit geleitet werden.

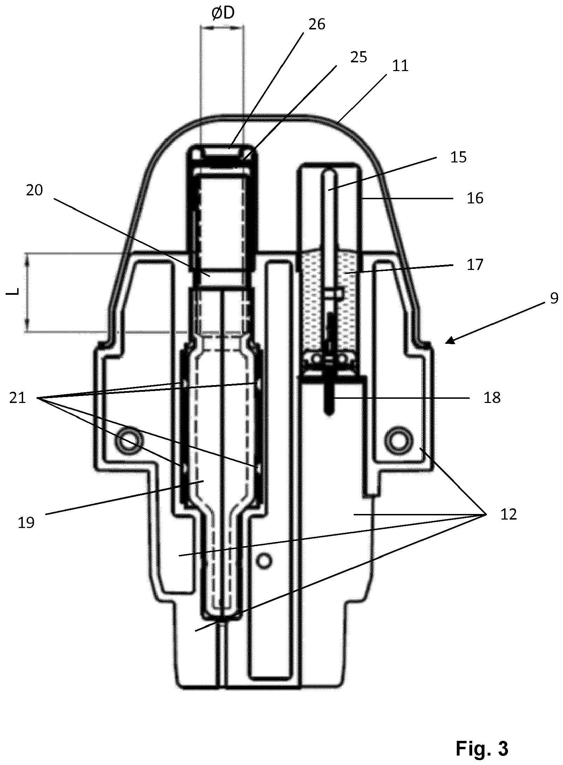

Der in Figur 3 im Querschnitt schematisch dargestellte Messkopf 9 zeigt ein Thermoelement als Temperatursensor 15, der von einer Metallkappe 16 umgeben und mittels eines feuerfesten Zements 17 in dem Messkopf 9 angeordnet ist. An seinem rückwärtigen, im Inneren des Messkopfes 9 gelegen Ende weist er einen Verbinder 18 für den Anschluss der Thermoelementdrähte an das Verbindungskabel auf. In dem Sandkörper 12 aus Gießereisand des Messkopfes 9 ist ferner ein Probennehmer mit einer Probenkammer 19 und einem Quarzglasrohr 20 als Einlaufrohr angeordnet. Das Quarzglasrohr ragt etwa 1cm aus dem Sandkörper hinaus. Die äußere Einlauföffnung des Quarzglasrohres 20 ist mit einer Metallkappe 25 (aus Stahl) und einer darüber angeordneten Pappkappe 26 geschlossen, die bei oder nach Eintauchen in die Stahlschmelze zerstört werden und die äußere Einlauföffnung der Quarzglasrohres freigeben. Die Länge L bezeichnet die in dem Sandkörper 12 des Messkopfes 9 angeordnete Länge des Einlaufrohres zwischen seinem Eintritt in die Probenkammer 19 und seinem Austritt aus dem Sandkörper 12. Dies ist die sogenannte Einbaulänge L. Der Durchmesser D bezeichnet den Minimaldurchmesser innerhalb der Einbaulänge L. Im gezeigten Beispiel ist das Verhältnis L/D² = 0,22 und führt zu einer blasenfreien Probe, während es bei entsprechenden Sonden aus dem Stand der Technik bei etwa 1,43 liegt.

Die Probenkammer 19 ist durch Sandrippen 21 des Sandkörpers 12 in diesem durch Presspassung fixiert.

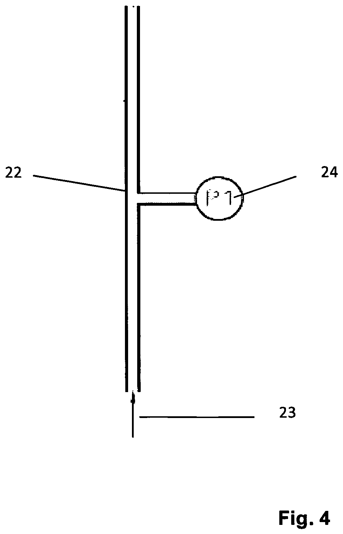

Die Druckmessung erfolgt zunächst gemäß der schematischen Darstellung in Figur 4 in einem beidseitig offenen Rohr 22, welches einen solchen Außendurchmesser aufweist, dass es in das Quarzglasrohr 20 eingeschoben werden kann. Der Pfeil 23 markiert die Flussrichtung des durchströmenden Gases, vorzugsweise Luft, dessen Druck P₁ mittels des Druckmessers 24 bestimmt wird. Die Länge des Rohres 22 zwischen dem Druckmesser 24 und dem Quarzglasrohr 20 ist etwa 2 cm, die Innendurchmesser ist etwa 4 mm.

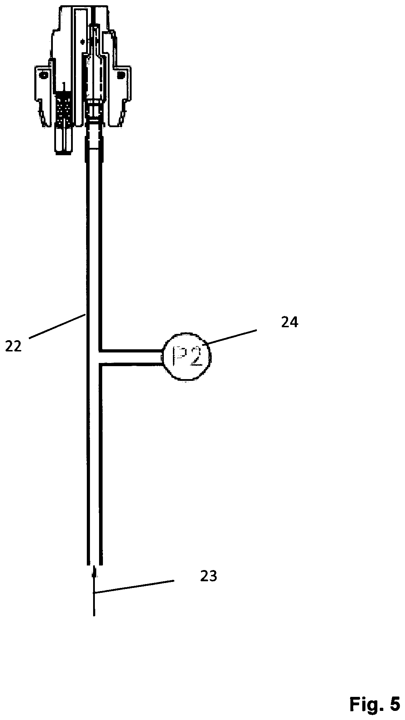

In Figur 5 ist das nach Messung gemäß Figur 4 in das Quarzglasrohr 20 des Probennehmers eingeschobene Rohr 22 schematisch dargestellt. Bei erneuter Gaseinleitung wird dann mittels des Druckmessers der Druck P₂ gemessen. Die Differenz P₂ - P₁ bezeichnet den Gegendruck P_{g} des Messkopfes. Der Druck wird jeweils gemessen bei Durchströmen eine Gases mit einer Flussrate von 800 l/h, wobei der Gasströmung ein sogenannter "Normalliter" zugrunde liegt, also gemessen bei einer Raumtemperatur von 20°C und einem Standardluftdruck von 1013 hPa. Der ermittelte Gegendruck beträgt im gezeigten Beispiel weniger als 15 mbar. Bei einer Anordnung mit diesem Gegendruck werden qualitativ gute Proben erzielt.

## Patentansprüche

1. Messsonde (8) zur Messung und Probennahme in einer Metallschmelze mit einem an einer Lanze angeordneten Messkopf (9), wobei der Messkopf mindestens einen Temperatursensor (15) und eine Probenkammer (19) trägt, wobei die Probenkammer zumindest teilweise von dem Messkopf umgeben ist und einen durch den Messkopf hindurch verlaufenden Einlaufkanal (20) aufweist, wobei der Einlaufkanal einen in dem Messkopf verlaufenden inneren Bereich mit einer Länge L aufweist und an mindestens einer Stelle in diesem inneren Bereich einen Mindestdurchmesser D hat, **dadurch gekennzeichnet, dass** das Verhältnis L/D² kleiner als 0,6 mm⁻¹ ist.

2. Messsonde nach Anspruch 1, **dadurch gekennzeichnet, dass** das Verhältnis L/D² kleiner als 0,45 mm⁻¹ ist.

3. Messsonde nach Anspruch 2, **dadurch gekennzeichnet, dass** das Verhältnis L/D² kleiner als 0,3 mm⁻¹ ist.

4. Messsonde nach Anspruch 1, **dadurch gekennzeichnet, dass** das Verhältnis L/D² kleiner als 0,6 mm⁻¹ und im Messkopf ein Gegendruck Pg vorliegt, welcher kleiner als 20 mbar ist.

5. Messsonde nach Anspruch 1, **dadurch gekennzeichnet, dass** das Verhältnis L/D² kleiner als 0,45 mm⁻¹ und im Messkopf ein Gegendruck Pg vorliegt, welcher kleiner als 20 mbar ist.

6. Messsonde nach Anspruch 1, **dadurch gekennzeichnet, dass** das Verhältnis L/D² kleiner als 0,3 mm⁻¹ und im Messkopf ein Gegendruck Pg vorliegt, welcher kleiner als 20 mbar ist.

7. Messsonde nach mindestens einem der Ansprüche 1 bis 6, **dadurch gekennzeichnet, dass** der Messkopf aus einem Material aus der Gruppe Keramik, Zement, Stahl, Gießereisand gebildet ist.

8. Messsonde nach mindestens einem der Ansprüche 1 bis 7, **dadurch gekennzeichnet, dass** der Einlaufkanal aus einem Quarzglasrohr gebildet ist.

9. Messsonde nach mindestens einem der Ansprüche 1 bis 8, **dadurch gekennzeichnet, dass** die Probenkammer zumindest teilweise von einem Sandkörper aus Gießereisand umgeben ist.

10. Messsonde nach mindestens einem der Ansprüche 1 bis 9, **dadurch gekennzeichnet, dass** die Probenkammer in einer ersten und einer zweiten, jeweils senkrecht zueinander angeordneten Richtung jeweils eine größere Länge aufweist als in einer dritten, senkrecht zu der ersten und zu der zweiten Richtung angeordneten Richtung und dass der Einlaufkanal senkrecht zu der dritten Richtung in die Probenkammer mündet.

11. Messsonde nach mindestens einem der Ansprüche 1 bis 10, **dadurch gekennzeichnet, dass** der Messkopf zusätzlich mindestens einen elektrochemischen Sensor trägt.

12. Verwendung der Messsonde nach mindestens einem der Ansprüche 1 bis 11 zur Messung und Probennahme in einer in einem Konverter zur Stahlherstellung angeordneten Metallschmelze während eines Blasprozesses.

13. Verwendung der Messsonde nach mindestens einem der Ansprüche 1 bis 11 zur Messung und Probennahme in einer in einem Lichtbogenofen angeordneten Metallschmelze.

## Claims

1. Measuring probe (8) for measuring and sample taking in molten metal having a measuring head (9) arranged on a lance, whereby the measuring head bears at least one temperature sensor (15) and one sample chamber (19), whereby the sample chamber is surrounded, at least in part, by the measuring head and comprises an inlet channel (20) that extends through the measuring head, whereby the inlet channel has an inner area of length L that extends in the measuring head and has a minimum diameter D in at least in one place in said inner area, **characterised in that** the ratio of L/D² is less than 0.6 mm⁻¹.

2. Measuring probe according to claim 1, **characterised in that** the ratio of L/D² is less than 0.45 mm⁻¹.

3. Measuring probe according to claim 2, **characterised in that** the ratio of L/D² is less than 0.3 mm⁻¹.

4. Measuring probe according to claim 1, **characterised in that** the ratio of L/D² is less than 0.6 mm⁻¹ and **in that** a counter-pressure Pg of less than 20 mbar predominates in the measuring head.

5. Measuring probe according to claim 1, **characterised in that** the ratio of L/D² is less than 0.45 mm⁻¹ and **in that** a counter-pressure Pg of less than 20 mbar predominates in the measuring head.

6. Measuring probe according to claim 1, **characterised in that** the ratio of L/D² is less than 0.3 mm⁻¹ and **in that** a counter-pressure Pg of less than 20 mbar predominates in the measuring head.

7. Measuring probe according to at least one of the claims 1 to 6, **characterised in that** the measuring probe is made from a material from the group of ceramics, cement, steel, foundry sand.

8. Measuring probe according to at least one of the claims 1 to 7, **characterised in that** the inlet channel is made from a quartz glass tube.

9. Measuring probe according to at least one of the claims 1 to 8, **characterised in that** the sample chamber is surrounded, at least in part, by a sand body made of foundry sand.

10. Measuring probe according to at least one of the claims 1 to 9, **characterised in that** the length of the sample chamber is larger in a first and in a second direction, which each are arranged to be perpendicular with respect to each other, than the length in a third direction that is arranged to be perpendicular to the first and to the second direction, and **in that** the inlet channel mergers into the sample chamber perpendicularly with respect to the third direction.

11. Measuring probe according to at least one of the claims 1 to 10, **characterised in that** the measuring head, in addition, bears at least one electrochemical sensor.

12. Use of the measuring probe according to at least one of the claims 1 to 11 for measuring and sample taking, a blow moulding process, in molten metal that is arranged in a converter for steel production during.

13. Use of the measuring probe according to at least one of the claims 1 to 11 for measuring and sample taking in molten metal arranged in an electric arc furnace.

## Revendications

1. Sonde de mesure (8) pour la mesure et le prélèvement d'échantillons dans un bain de fusion avec une tête de mesure (9) disposée sur une lance, dans laquelle la tête de mesure porte au moins un capteur de température (15) et une chambre d'échantillons (19), dans laquelle la chambre d'échantillons est au moins partiellement entourée par la tête de mesure et présente un canal d'entrée (20) s'étendant à travers la tête de mesure, dans laquelle le canal d'entrée présente une région interne s'étendant dans la tête de mesure avec une longueur L et a à au moins un endroit dans cette région interne un diamètre minimal D, **caractérisée en ce que** le rapport L/D² est inférieur à 0,6 mm⁻¹.

2. Sonde de mesure selon la revendication 1, **caractérisée en ce que** le rapport L/D² est inférieur à 0,45 mm⁻¹.

3. Sonde de mesure selon la revendication 2, **caractérisée en ce que** le rapport L/D² est inférieur à 0,3 mm⁻¹.

4. Sonde de mesure selon la revendication 1, **caractérisée en ce que** le rapport L/D² est inférieur à 0,6 mm⁻¹ et une contrepression Pg est présente dans la tête de mesure, laquelle est inférieure à 20 mbar.

5. Sonde de mesure selon la revendication 1, **caractérisée en ce que** le rapport L/D² est inférieur à 0,45 mm⁻¹ et une contrepression Pg est présente dans la tête de mesure, laquelle est inférieure à 20 mbar.

6. Sonde de mesure selon la revendication 1, **caractérisée en ce que** le rapport L/D² est inférieur à 0,3 mm⁻¹ et une contrepression Pg est présente dans la tête de mesure, laquelle est inférieure à 20 mbar.

7. Sonde de mesure selon au moins une des revendications 1 à 6, **caractérisée en ce que** la tête de mesure est formée d'un matériau provenant du groupe céramique, ciment, acier, sable de fonderie.

8. Sonde de mesure selon au moins une des revendications 1 à 7, **caractérisée en ce que** le canal d'entrée est formé d'un tube en verre de quartz.

9. Sonde de mesure selon au moins une des revendications 1 à 8, **caractérisée en ce que** la chambre d'échantillons est au moins partiellement entourée par un corps de sable en sable de fonderie.

10. Sonde de mesure selon au moins une des revendications 1 à 9, **caractérisée en ce que** la chambre d'échantillons présente dans une première et une deuxième directions disposées chacune perpendiculairement l'une à l'autre à chaque fois une longueur supérieure à dans une troisième direction disposée perpendiculairement à la première et à la deuxième direction, et que le canal d'entrée débouche dans la chambre d'échantillons perpendiculairement à la troisième direction.

11. Sonde de mesure selon au moins une des revendications 1 à 10, **caractérisée en ce que** la tête de mesure porte en outre au moins un capteur électrochimique.

12. Utilisation de la sonde de mesure selon au moins une des revendications 1 à 11 pour la mesure et le prélèvement d'échantillons dans un bain de fusion disposé dans un convertisseur pour la fabrication d'acier pendant un processus de soufflage.

13. Utilisation de la sonde de mesure selon au moins une des revendications 1 à 11 pour la mesure et le prélèvement d'échantillons dans un bain de fusion disposé dans un four à arc indirect.
